# EUROPEAN PATENT APPLICATION

(11) **EP 4 778 507 A1**
(43) Date of publication of application: **22.07.2026**
(21) Application number: 24865891.6
(22) Date of filing: 12.09.2024
(51) Int. Cl.: A61K 8/42, A61Q 5/00, A61Q 5/12

(54) **COSMETIC COMPOSITION FOR REPAIRING DAMAGED HAIR**

(30) Priority: 12.09.2023 KR 20230121185
(71) Applicant: Daebong LS Co., Ltd., Namdong-gu Incheon 21697 (KR); UCL Co. Ltd., Jeju-si, Jeju-do 63038 (KR)
(72) Inventor: PARK, Eun Ju, Hwaseong-si Gyeonggi-do 18425 (KR); LEE, Seon Mi, Incheon 21439 (KR); CHOI, Ah Ram, Incheon 22020 (KR); LEE, Jeong Kyeong, Incheon 21675 (KR); BAEK, Seok Yun, Seongnam-si Gyeonggi-do 13611 (KR); LEE, Ji Won, Seoul 06276 (KR); PARK, Jin Oh, Seoul 06276 (KR)
(74) Representative: Ter Meer Steinmeister & Partner
(86) International application number: PCT/KR2024/013949
(87) International publication number: WO 2025/058452

(57) **Abstract**

The present invention relates to a cosmetic composition for hair, comprising a bis (haloacetamide)-PEG compound as an active ingredient, wherein the cosmetic composition is safe for cosmetic use, repairs damaged hair, and can be used in the form of a formulation such as shampoo, treatment, serum, cream, oil, and the like. More specifically, the present invention relates to a cosmetic composition for hair comprising a compound represented by chemical formula 1 below as an active ingredient. When a hair protecting composition of the present invention is mixed with general hair lotion, bonds of hair broken due to various chemical procedures are recombined, and an effect of restoring smoothness, gloss, tensile strength, and the like of hair can be expected. In chemical formula 1, X is halogen, and n is an integer of 1-10.

## Description

### [Technical Field]

The present invention relates to a cosmetic composition for hair comprising a bis (haloacetamide)-PEG (eg., a bis-bromoacetylaminoethyl glycol) compound as an active ingredient, and more particularly, to a cosmetic composition that is safe for cosmetic use, repairs damaged hair and can be used as various forms such as a shampoo, treatment, serum, cream, oil, or the like.

### [Background Art]

Hair has a fibrous bundle structure in which approximately 18 types of amino acids are repeatedly linked through peptide bonds to form millions of polypeptides that are regularly twisted. The peptide bonds form the main chain structure and provide high mechanical strength, and the hair structure is further stabilized laterally by disulfide bonds, salt bonds, and hydrogen bonds.

The salt bond is also referred to as an "ionic bond", is an intermolecular ionic interaction formed between negatively charged acidic amino acids having carboxyl groups in the side chains thereof, such as aspartic acid and glutamic acid, and positively charged basic amino acids having two amino groups and one carboxyl group, such as lysine, arginine, and histidine. Such ionic bonds exhibit relatively weak bonding strength. The salt bonds are easily disrupted by changes in pH and are restored when the pH returns to normal. Although salt bonds are weaker than disulfide bonds, the large number of salt bonds correspons to approximately 30% of the overall strength of hair.

Hydrogen bonds are very weak physical transverse bonds formed due to differences in electronegativity between hydrogen and oxygen atoms, and are readily broken by water or heat but are re-formed upon drying or cooling.

A disulfide bond is a very strong bond called an "S-S bond", which is a covalent bond between sulfur atoms in the form of -S-S-, where the thiol groups of two adjacent cysteines are bonded together.

However, even such strong disulfide bonds may be easily modified or damaged by intense heat or chemical treatments such as permanent waving or hair dyeing. In other words, these side chain bonds are readily affected by chemical actions and are cleaved by chemical treatments including permanent waving, dyeing, and bleaching, resulting in the loss of portions of protein and ultimately causing hair damage.

The side chain bonds that determine the elasticity and strength of hair exhibit reversible properties, repeatedly breaking and reforming depending on water, pH, and chemical oxidation/reduction reactions. However, repeated chemical treatment, severe heat treatment, or harsh chemical exposure may cause partial loss of these bonds, preventing recombination and leading to permanent hair damage.

Conventional technologies directed to cosmetic compositions for restoring hair from such permanent damage include, for example, Korean Patent Publication No. 2021-0081672 (Patent Document 1). The disclosed invention relates to a cosmetic composition for recovering damaged hair that contains, based on the total weight of the composition, 75 to 94 wt% of a keratin protein or peptide having a molecular weight of 520 to 3,000 Daltons, 5 to 20 wt% of a coconut oil, and 1 to 5 wt% of citric acid, and thus has effects such as glossiness, lubricity, moisturization, and styling elasticity.

However, the addition of keratin protein or peptide, which is a merely structural component of hair, makes it difficult to expect recovery of side chain bonds such as disulfide bonds, and thus the hair restoration effect is very limited.

Accordingly, there remains an urgent need for research on cosmetic compositions capable of restoring physically, chemically, or environmentally damaged hair to its original healthy state.

### [Prior Art Document]

### [Patent Document]

(Patent Document 1) KR 10-2021-0081672 A (July 2, 2021)

### [Disclosure]

### [Technical Problem]

Therefore, the present invention has been made in view of the above problems, and it is an object of the present invention to provide a composition that prevents hair damage by providing a binding material to hair damaged by frequent dyeing, bleaching, and perming, and restores hair to healthy condition by strengthening the internal fiber tissue of the hair.

The various objects to be achieved by the present invention are not limited to the above-mentioned objects, and other objects not specifically mentioned herein will be clearly understood by those skilled in the art from the following description.

### [Technical Solution]

In accordance with the present invention, the above and other objects can be accomplished by the provision of
a hair cosmetic composition containing, as an active ingredient, a compound represented by the following Formula 1: 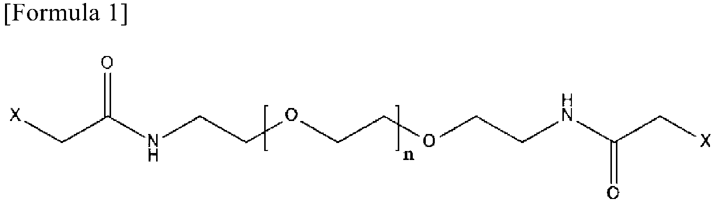
wherein X is a halogen and n is an integer of 1 to 10.

In one embodiment of the present invention, the compound of Formula 1 may be represented by the following Formula 2:

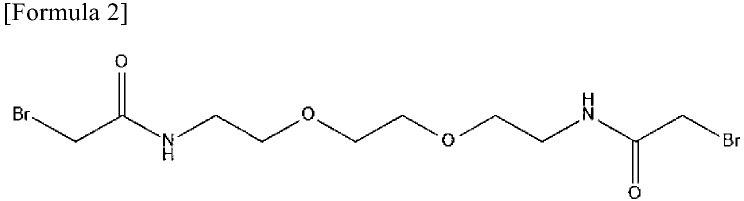

In another embodiment, the compound of Formula 1 may be represented by the following Formula 3:

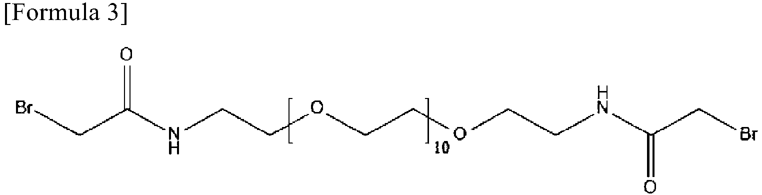

In an embodiment, the active ingredient may be present in an amount of 0.001 to 10 wt% based on the total weight of the composition.

In an embodiment, the composition may further contain a foaming agent, a cleansing agent, a conditioning agent, a pH adjusting agent, a pearlizer, a preservative, a fragrance, a viscosity modifier, a stabilizer, a moisturizing agent, a thickener, a lower alcohol, a chelating agent, or a colorant.

In an embodiment, a formulation for the composition may include at least one of a hair lotion, a hair serum, a hair essence, a hair tonic, a hair nutrient solution, a shampoo, a rinse, a conditioner, a hair treatment, a permanent wave agent, a hair styling product, a hair mousse, a hair bleach, a hair coloring product, or an oxidative hair coloring product.

### [Advantageous effects]

When mixed with a conventional hair lotion, the hair protective composition of the present invention may have effects of promoting reformation of broken hair bonds caused by various chemical treatments and of restoring smoothness, gloss, and tensile strength of the hair.

The effects of the present invention are not limited to those described above and should be construed as including all effects that can be reasonably inferred from the following description.

### [Description of Drawings]

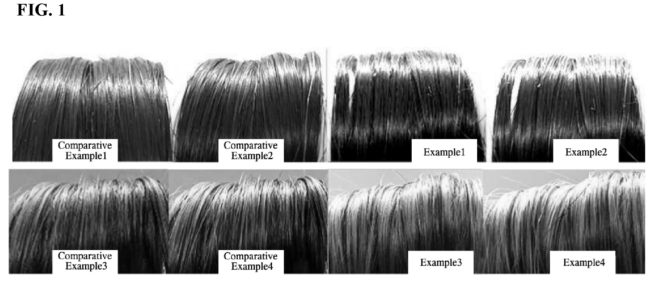

FIG. 1 is an image showing the results of a gloss test of the present invention.

### [Best Mode]

Hereinafter, the present invention will be described in detail.

In one aspect, the present invention is directed to a hair cosmetic composition containing, as an active ingredient, a compound represented by the following Formula 1: 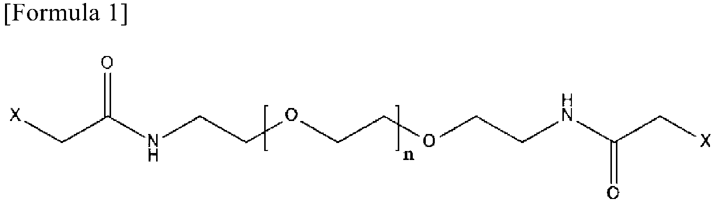 wherein X is a halogen and is selected from fluorine, chlorine, bromine, or iodine, preferably, is chlorine, bromine, or iodine, more preferably bromine or iodine, and most preferably bromine. The selection of the halogen may take into account various factors, including reactivity, toxicity of the reaction product, and color.

Furthermore, n is an integer ranging from 1 to 10, preferably 1 to 8, more preferably 1 to 5, and even more preferably 1 to 3. n determines the chain length and may also affect water solubility. Accordingly, those skilled in the art may appropriately adjust the composition in consideration of various factors, such as the flowability, adhesion, permeability, reactivity, formulation type, handleability, degree of hair damage, duration of hair damage, and cost-effectiveness of the formulation.

As a specific example, the compound of Formula 1 may be represented by Formula 2 or Formula 3.

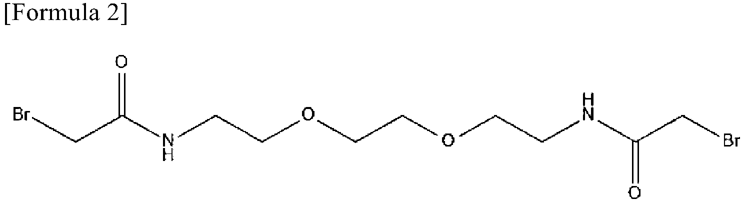

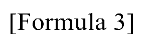

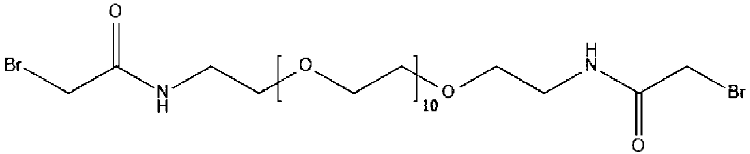

The active ingredient may repair damaged hair bonds by reformation thereof. A more detailed understanding of this may be achieved from the examples and experimental tests described below.

The active ingredient is present in an amount of 0.001 to 10 wt%, preferably, 0.01 to 5 wt%, based on the total weight of the composition. Below the above range, the intended effects may be achieved due to excessively small content. Above the range, there may be difficulties in formulation.

In addition to the active ingredient, the composition may further contain a foaming agent, a cleansing agent, a conditioning agent, a pH adjusting agent, a pearlizer, a preservative, a fragrance, a viscosity modifier, a stabilizer, a moisturizing agent, a thickener, a lower alcohol, a chelating agent, or a colorant.

The foaming agent or cleansing agent may contain an anionic surfactant, a zwitterionic or amphoteric surfactant, a nonionic surfactant, or a combination thereof.

The anionic surfactant may be selected from the group consisting of alkyl sulfates, alkyl sulfonates, alkyl carboxylates, alkyl ether sulfates, alkyl isethionates, alkyl taurates, alkyl sulfosuccinates, alkyl sulfoacetates, alkyl phosphates, anionic amino acid derivatives, anionic polypeptide derivatives, anionic derivatives of alkyl polyglucosides, fatty acid soaps, and mixtures thereof. Representative examples of the anionic surfactant include ammonium lauryl sulfate (ALS), ammonium laureth sulfate (ALES), sodium lauryl sulfate (SLS), and sodium laureth sulfate (SLES). The anionic surfactant may be present alone or in combination of two or more in an amount of 1 to 25 wt%, preferably 5 to 20 wt%, based on the total weight of the composition.

In addition, zwitterionic or amphoteric surfactants are not particularly limited and examples thereof may include (C8-C20)alkyl betaines, N-(C8-C20) alkylamidobetaines and derivatives thereof, glycine derivatives, sultaines and derivatives thereof, (C8-C20) alkyl polyaminocarboxylates, (C8-C20)alkyl amphoacetates, sodium lauroamphoacetate, and mixtures thereof. Preferably, for example, zwitterionic or amphoteric surfactants are selected from the group consisting of amine oxides, cocoamphocarboxyglycinate, cocamidopropyl betaine, and mixtures thereof. The content of the zwitterionic or amphoteric surfactant is preferably less than 10 wt% based on the total weight of the composition.

The nonionic surfactant may be selected from block copolymers of poly(ethylene oxide) and poly(propylene oxide), alkyl and polyalkyl esters of poly(ethylene oxide), oxyalkylenated alcohols, alkyl and polyalkyl ethers of poly(ethylene oxide), alkyl and polyalkyl esters of polyoxyethylene sorbitan, alkyl and polyalkyl ethers of polyoxyethylene sorbitan, alkyl or polyalkyl glycosides or polyglycosides, alkyl and polyalkyl esters of sucrose, alkyl and polyalkyl ethers of glycerol polyoxyethylene, gemini surfactants, alkyl alcohol-based surfactants, and mixtures thereof. The nonionic surfactant is preferably present in an amount of less than 10 wt% based on the total weight of the composition.

The conditioning agent may be selected from the group consisting of dimethicone-based materials, hydrogenated polydecene, and cationic polymers. The conditioning agent may be used alone or in combination of two or more in an amount of 0.1 to 10 wt%, preferably 0.5 to 5 wt%, based on the total weight of the composition.

The pH adjusting agent is not particularly limited and examples thereof may include sulfuric acid, hydrochloric acid, phosphoric acid, acetic acid, lactic acid, sodium phosphate, disodium phosphate, citric acid and sodium citrate. The pH adjusting agent is preferably used alone or in combination of two or more in an amount of 0.1 to 2 wt% based on the total weight of the composition.

The pearlizer may include glycol distearate, glycol monostearate, fatty acids, or the like.

The preservative may include methyl parahydroxybenzoate, propyl parahydroxybenzoate, sodium benzoate, methylchloroisothiazolinone, methylisothiazolinone, or a mixture thereof.

The viscosity modifier may include an amide-based nonionic surfactant such as cocamide MEA, cocamide DEA, or sodium chloride.

The moisturizing agent is not particularly limited and may include glycerin, propylene glycol, 1,3-butylene glycol, dipropylene glycol, sorbitol, and the like.

The thickener is not particularly limited and may include water-soluble polymer compounds such as methyl cellulose, hydroxyethyl cellulose, carrageenan, carboxymethyl cellulose, hydroxymethyl cellulose, xanthan gum and derivatives thereof, guar gum and derivatives thereof, acacia gum and derivatives thereof, arabic gum and derivatives thereof, Carbopol^{®} and derivatives thereof, and mixtures thereof. The colorant may include any conventional colorants commonly used in scalp or hair formulations without particular limitation.

In another aspect, a conditioner containing the hair cosmetic composition according to the present invention may further contain at least one appropriate ingredient suitable for the type of conditioner. For example, conventional conditiner ingredients include cationic surfactants such as tertiary amidoamines and quaternary ammonium compounds, high-melting-point compounds, silicone compounds, and optional components such as preservatives, thickeners, viscosity modifiers, pH adjusters, and fragrances.

In the conditioner containing the hair cosmetic composition, quaternary ammonium compounds as cationic surfactants may include may include alkyl (C14-C22) trimethyl ammonium chloride, dialkyl (C14-C22) dimethyl ammonium chloride, hydrogenated tallow alkyl trimethyl ammonium chloride, and ditallow alkyl dimethyl ammonium chloride. For example, the tertiary amidoamines may include cocamidopropyl dimethylamine, stearamidopropyl dimethylamine, behenamidopropyl dimethylamine, oleamidopropyl dimethylamine, and isostearamidopropyl dimethylamine. In addition, the amines are preferably neutralized with an acid such as L-glutamic acid, lactic acid, hydrochloric acid, malic acid, succinic acid, acetic acid, fumaric acid, citric acid, or EDTA. In addition, the cationic surfactant may be used alone or in combination of two or more in an amount of 1 to 30 wt%, preferably 5 to 20 wt%, based on the total weight of the composition.

The high-melting-point compounds may include fatty alcohols, fatty acids, fatty alcohol derivatives, hydrocarbons, and mixtures thereof, such as cetyl alcohol, stearyl alcohol, and cetearyl alcohol. The content thereof may be 1 to 10 wt% based on the total weight of the conditioner. The silicone compound may include polyalkyl siloxanes, polyaryl siloxanes, polyalkylaryl siloxanes, polyether siloxane copolymers and mixtures thereof, cyclopolysiloxanes, alkylamino-substituted silicones, amodimethicone, and the like. The content thereof may be 1 to 10 wt% based on the total weight of the conditioner.

Optional ingredients may include conventional additives widely known in the art for maintaining basic physical properties and quality, and specific examples thereof include preservatives, thickeners, viscosity modifiers, pH adjusting agents, fragrances, stabilizers, moisturizing agents, lower alcohols, chelating agents, and colorants.

The composition may be prepared in various formulations including, but not limited to, hair lotions, hair serums, hair essences, hair tonics, hair nutrient solutions, shampoos, rinses, conditioners, hair treatments, permanent wave agents, hair styling products, hair mousses, hair bleaches, hair coloring products, and oxidative hair coloring products. However, the formulation is not particularly limited thereto and may include any conventional formulation known in the art.

Hereinafter, the present invention will be described in more detail with reference to the following examples. However, these examples are provided for illustrative purposes only and are not intended to limit the scope of the present invention in any manner.

### Example

**[Table 1]**

| | Ingredient name | Comparative Example 1 | Comparative Example 2 | Example 1 | Example 2 | Comparative Example 3 | Comparative Example 4 | Example 3 | Example 4 |
|---|---|---|---|---|---|---|---|---|---|
| 1 | Bis(bromoacetami de)-PEG2 | - | 0 | 0.1 | 0.5 | - | 0 | 0.1 | 0.5 |
| 2 | Glycerin | - | 5 | 5 | 5 | - | 5 | 5 | 5 |
| 3 | Potassium sorbate | - | 0.03 | 0.03 | 0.03 | - | 0.03 | 0.03 | 0.03 |
| 4 | Cetrimonium chloride | - | 1.5 | 1.5 | 1.5 | - | 1.5 | 1.5 | 1.5 |
| 5 | Behentrimonium methosulfate | - | 1.5 | 1.5 | 1.5 | - | 1.5 | 1.5 | 1.5 |
| 6 | Cedearyl alcohol | - | 4.0 | 4.0 | 4.0 | - | 4.0 | 4.0 | 4.0 |
| 7 | Cetyl alcohol | - | 4.5 | 4.5 | 4.5 | - | 4.5 | 4.5 | 4.5 |
| 8 | Behenyl alcohol | - | 2.0 | 2.0 | 2.0 | | 2.0 | 2.0 | 2.0 |
| 9 | Stearamidopropyl dimethylamine | - | 1.0 | 1.0 | 1.0 | - | 1.0 | 1.0 | 1.0 |
| 10 | Sunflower seed oil | - | 5.0 | 5.0 | 5.0 | - | 5.0 | 5.0 | 5.0 |
| 11 | Purified water | 100 | To 100 | To 100 | To 100 | 100 | To 100 | To 100 | To 100 |
| | | Healthy hair | Healthy hair | Healthy hair | Healthy hair | Damaged hair | Damaged hair | Damaged hair | Damaged hair |

Comparative Examples 1 to 4 and Examples 1 to 4 were mixed with the ingredients listed in Table 1 in a mixing container and stirred for 5 minutes using a homomixer. The following experiments were conducted.

### Experimental Example

### 1. Conditioning Evaluation

To evaluate the conditioning of Comparative Examples 1 to 4 and Examples 1 to 4, the friction force of experimental hair having a weight of 6 g and a length of 100 mm was measured using a friction coefficient tester (Neo-Tribo (FCMS 170)). Each sample was evenly applied to the same hair.

At this time, 2 g each of test samples of Comparative Examples 1 and 2 and Examples 1 and 2 were applied to 6 g of healthy hair tufts and allowed to be absorbed, and 2 g each of the test samples of Comparative Examples 3 and 4 and Examples 3 and 4 were applied to 6 g of damaged hair tufts that had been bleached three times and allowed to be absorbed, thereby treating the samples (i.e., healthy hair is the untreated group, damaged hair is the bleached group, and each hair tuft may be the same or different origins).

The frictional force of the dried hair was measured using a friction coefficient tester (Neo-Tribo (FCMS 170)).

A larger change in the friction coefficient indicates a better hair conditioning effect.

**[Table 2]**

| Friction | Comparati | Comparati | Example1 | Example 2 | Comparati | Comparati | Example 3 | Example 4 |
|---|---|---|---|---|---|---|---|---|
| coefficient | ve Example1 | ve Example2 | | | ve Example3 | ve Example4 | | |
| 1st | 0.175 | 0.147 | 0.158 | 0.044 | 0.143 | 0.138 | 0.139 | 0.053 |
| 2nd | 0.157 | 0.152 | 0.154 | 0.043 | 0.139 | 0.142 | 0.141 | 0.052 |
| 3rd | 0.158 | 0.157 | 0.144 | 0.043 | 0.138 | 0.148 | 0.138 | 0.043 |
| Average | 0.163 | 0.152 | 0.152 | 0.043 | 0.140 | 0.143 | 0.139 | 0.049 |

### 2. Evaluation of hair gloss (shine) effect

To evaluate the hair gloss (shine) effects of Comparative Examples 1 to 4 and Examples 1 to 4, the test samples were evenly applied to hair samples and dried (i.e., healthy hair was the untreated group, damaged hair was the bleached group, and each hair tuft could have the same or different origins).

The hair sample was exposed to constant illumination, and a gloss band formed by light scattering on the hair surface was visually evaluated. An increase in the band area and uniformity of the gloss band were considered to indicate enhanced glossiness of the hair.

The results are shown in FIG. 1 and Table 3 below.

**[Table 3]**

| Comparative Example1 | Comparative Example2 | Example1 | Example2 | Comparative Example 3 | Comparative Example 4 | Example3 | Example 4 |
|---|---|---|---|---|---|---|---|
| ++ | ++ | +++ | ++++ | | ++ | +++ | +++ |
| Note: The symbol "+" represents the degree of gloss evaluated using a five-point rating scale by the researcher, and a greater number of "+" indicates that a wider gloss band was observed. | | | | | | | |

### 3. Evaluation of damged hair bonding

In order to evaluate the bonding of damaged hair and the elasticity of the hair according to Comparative Examples 1 to 4 and Examples 1 to 4, a tensile strength test was conducted as follows.

2 g of each test sample of Comparative Examples 1 to 2 and Examples 1 to 2 was applied to 6 g of healthy hair tufts and allowed to be absorbed. 2 g of each test sample of Comparative Examples 3 to 4 and Examples 3 to 4 was applied to 6 g of damaged hair tufts that had been bleached three times and allowed to be absorbed, thereby treating the samples (i.e., healthy hair is the untreated group, damaged hair is the bleached group, and each hair tuft may be the same or different origins).

The tensile strengths of the dried hair samples were measured using a tensile strength tester (model: Neo-Tribo (FCMS 170)). The measurements were repeated ten times and the obtained values were statistically analyzed.

The results are shown in Table 4 below.

**[Table 4]**

| Tensile strength (g/cm²) | Comparative Example1 | Comparative Example2 | Example 1 | Example2 | Comparative Example3 | Comparative Example4 | Example3 | Example4 |
|---|---|---|---|---|---|---|---|---|
| 1 | 0.311 | 0.419 | 0.504 | 0.573 | 0.399 | 0.748 | 0.870 | 1.001 |
| 2 | 0.260 | 0.518 | 0.495 | 0.500 | 0.654 | 0.574 | 0.852 | 0.954 |
| 3 | 0.421 | 0.588 | 0.461 | 0.779 | 0.654 | 0.632 | 0.687 | 0.885 |
| 4 | 0.222 | 0.594 | 0.576 | 0.593 | 0.486 | 0.614 | 0.774 | 0.909 |
| 5 | 0.303 | 0.297 | 0.494 | 0.484 | 0.666 | 0.736 | 0.710 | 0.755 |
| 6 | 0.194 | 0.295 | 0.694 | 0.449 | 0.504 | 0.761 | 0.631 | 0.907 |
| 7 | 0.464 | 0.523 | 0.529 | 0.533 | 0.56 | 0.698 | 0.618 | 0.895 |
| 8 | 0.256 | 0.537 | 0.443 | 0.476 | 0.599 | 0.483 | 0.640 | 0.841 |
| 9 | 0.377 | 0.555 | 0.577 | 0.531 | 0.481 | 0.648 | 0.663 | 0.793 |
| 10 | 0.312 | 0.234 | 0.511 | 0.454 | 0.612 | 0.507 | 0.889 | 0.873 |
| Average | **0.312** | **0.456** | **0.5284** | **0.5372** | **0.5615** | **0.6401** | **0.7334** | **0.8813** |

As can be seen from the experiment, the hair cosmetic composition according to the present invention promotes reformation of damaged hair bonds, thereby restoring hair elasticity and repairing hair damage. In addition, the composition improves the smoothness and gloss of the hair.

### [Industrial Applicability]

The present invention is industrially applicable in the field of hair care.

## Claims

1. A hair cosmetic composition comprising a compound represented by the following Formula 1 as an active ingredient: 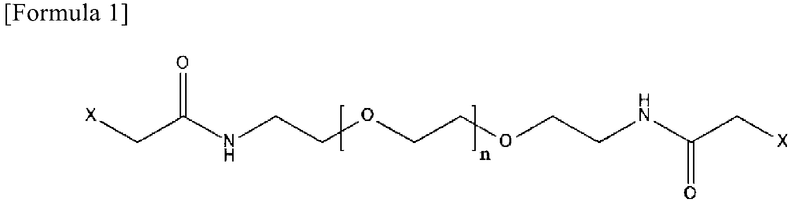 wherein X is a halogen and n is an integer of 1 to 10.

2. The hair cosmetic composition according to claim 1, wherein the compound of Formula 1 is represented by the following Formula 2:

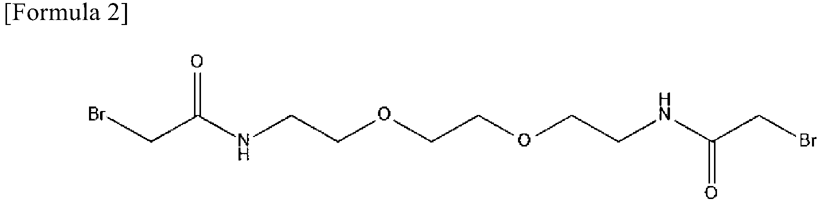

3. The hair cosmetic composition according to claim 1, wherein the compound of Formula 1 is represented by the following Formula 3:

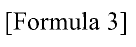

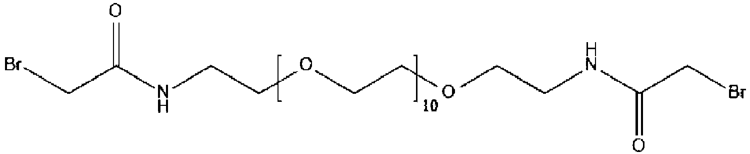

4. The hair cosmetic composition according to claim 1, wherein the active ingredient is present in an amount of 0.001 to 10 wt% based on the total weight of the composition.

5. The hair cosmetic composition according to claim 1, wherein the composition further comprises a foaming agent, a cleansing agent, a conditioning agent, a pH adjusting agent, a pearlizer, a preservative, a fragrance, a viscosity modifier, a stabilizer, a moisturizing agent, a thickener, a lower alcohol, a chelating agent, or a colorant.

6. The hair cosmetic composition according to any one of claims 1 to 5, wherein a formulation for the composition comprises at least one of a hair lotion, a hair serum, a hair essence, a hair tonic, a hair nutrient solution, a shampoo, a rinse, a conditioner, a hair treatment, a permanent wave agent, a hair styling product, a hair mousse, a hair bleach, a hair coloring product, or an oxidative hair coloring product.
